# FASCICULE DE BREVET EUROPEEN

(11) **EP 0 630 633 B1**
(45) Date de publication et mention de la délivrance du brevet: **26.07.2000**
(21) Numéro de dépôt: 94460015.4
(22) Date de dépôt: 14.06.1994
(51) Int. Cl.: A61F 15/00

(54) **Dispositif de protection étanche pour un moyen de contention**
Undurchlässige Schutzvorrichtung für einen Stützverband
Impermeable protective device for a supporting bandage

(30) Priorité: 16.06.1993 FR 9307357
(43) Date de publication de la demande: 28.12.1994
(73) Titulaire: Lanthier, Catherine, épouse Dosdat, F-53320 Loiron (FR); CHIRURGIE OUEST SA, 35769 Saint-Grégoire Cédex (FR)
(72) Inventeur: Lanthier, Catherine, F-53320 Loiron (FR)
(74) Mandataire: Maillet, Alain

(56) Documents cités:
- WO-A-93/14730
- DE-U- 8 911 168
- FR-A- 2 510 885
- US-A- 4 346 699

## Description

La présente invention concerne un dispositif de protection étanche pour un moyen de contention utilisé après un traumatisme quelqu'en soit l'origine et porté par un membre supérieur ou inférieur. Un tel dispositif de protection est plus particulièrement destiné à protéger ledit moyen de contention ou de recouvrement au cours de douches et de bains que le patient prend.

Bien que les contentions en résine soient immergeables, elles obligent le patient à sécher ensuite la surface cutanée, en général à l'air chaud, par exemple au moyen d'un sèche-cheveux, pour éviter les risques de macération cutanée. Il y a donc un besoin pour un dispositif de protection tel que défini ci-dessus.

Actuellement, cette protection étanche d'un moyen de contention est assurée par des moyens rudimentaires et peu efficaces ou, au contraire, trop sophistiqués rendant son utilisation ardue et sa fabrication onéreuse. On citera, à titre d'exemple, le dispositif de protection des plâtres décrit dans le document de brevet FR-A-2 650 949 qui nécessite un gonflage de la partie qui assure l'étanchéité.

On connaît également, par le document de brevet DE-A-89 11 168, un dispositif de protection étanche pour un moyen de contention, tel qu'un pansement ou un plâtre, porté par un membre supérieur ou inférieur, qui comprend un manchon destiné à être enfilé sur ledit membre et donc à le protéger. Ledit manchon est pourvu, en prolongement de son ou de ses extrémités ouvertes, de moyens élastiques d'application sur le membre.

Dans ce document, les moyens élastiques d'application sont constitues d'une bande élastique de diamètre constant sur toute sa largeur qui est montée à l'extrémité ouverte du manchon.

Or, pour assurer une bonne étanchéité entre le dispositif lui-même et le membre qui porte le moyen de contention, cette bande élastique doit présenter un diamètre relativement plus faible que le diamètre de la partie de membre sur laquelle elle s'applique. Il s'ensuit que, dans la plupart des cas, elle garrotte cette partie, effet qui est plus ou moins important selon la grosseur dudit membre et qui n'est pas souhaitable.

Par ailleurs, du fait de ce faible diamètre de la bande élastique, le dispositif de protection décrit dans ce document s'avère relativement difficile à mettre en place sur le moyen de contention.

On connaît également par le document de brevet américain US-A-4 346 699 un dispositif de protection du type qui est constitué d'une enveloppe protectrice globalement cylindrique, dont une portion d'extrémité est en forme de cou. Ladite portion d'extrémité présente également un diamètre constant, de sorte à comprimer le membre concerné sur toute sa surface latérale. Par conséquent, on observe l'effet de garrot précité.

On connaît en outre par le document de brevet français FR-A-2 510 885 un autre dispositif de protection constitué d'une enveloppe dont chaque portion terminale est de diamètre constant et inférieur à celui du reste de ladite enveloppe. Chaque portion terminale est par exemple logée dans un ourlet formé en l'une des parties d'extrémité de ladite enveloppe, et elle est prévue pour assurer un placage efficace de l'enveloppe sur le membre concerné.

De même que précédemment, il s'ensuit un garrottage du membre aux extrémités de ladite enveloppe.

Le but de l'invention est donc de proposer un dispositif de protection du type comportant un manchon qui est destiné à être enfilé sur un membre, ledit dispositif étant prévu pour être appliqué d'une manière élastique sur ledit membre par sa ou ses extrémités ouvertes, ladite ou lesdites extrémités ouvertes présentant un diamètre inférieur à celui dudit membre, qui remédie à ces problèmes, c'est-à-dire qui n'engendre pas d'effet de garrot et qui soit plus aisé à enfiler que les dispositifs connus.

A cet effet, un dispositif de protection selon l'invention est caractérisé en ce que ledit dispositif comporte au moins un anneau élastique en forme de couronne, ledit ou chaque anneau étant solidarisé par son extrémité externe par rapport audit membre avec ledit manchon, ledit ou chaque anneau présentant, d'une part, un diamètre maximal au niveau de ladite extrémité externe et, d'autre part, un diamètre minimal seulement au niveau de son extrémité interne par rapport audit membre, ladite extrémité interne formant ladite ou chaque extrémité ouverte dudit dispositif.

Selon un mode de réalisation préféré de l'invention, ledit ou chaque anneau élastique est cousu sur une extrémité ouverte du manchon. Il peut également être collé ou soudé.

Afin d'améliorer l'étanchéité entre le manchon et l'anneau, ladite extrémité externe dudit ou de chaque anneau et une extrémité ouverte du manchon sont montées bord à bord et sont recouvertes d'un ruban de section en forme de "U".

Avantageusement, ladite extrémité externe dudit ou de chaque anneau, ladite extrémité ouverte du manchon et ledit ruban sont solidarisés par couture, ou surpiquage. Ils peuvent également être solidarisés par collage ou par soudage.

L'anneau élastique est avantageusement en latex. Quant au manchon, il est constitué d'une enveloppe en matière plastique ou en matériau tissé ou non-tissé.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels:
la Fig. 1 est une vue en perspective d'un dispositif de protection selon l'invention disposé sur un membre traumatisé portant un plâtre,
la Fig. 2 est une vue en élévation d'un dispositif selon l'invention,
la Fig. 3 est une vue en plan d'un anneau élastique qui constitue un élément du dispositif de protection selon l'invention,
la Fig. 4 illustre le fonctionnement de l'anneau élastique du dispositif de protection selon l'invention, et
la Fig. 5 est une vue en coupe selon un plan médian d'un dispositif selon l'invention.

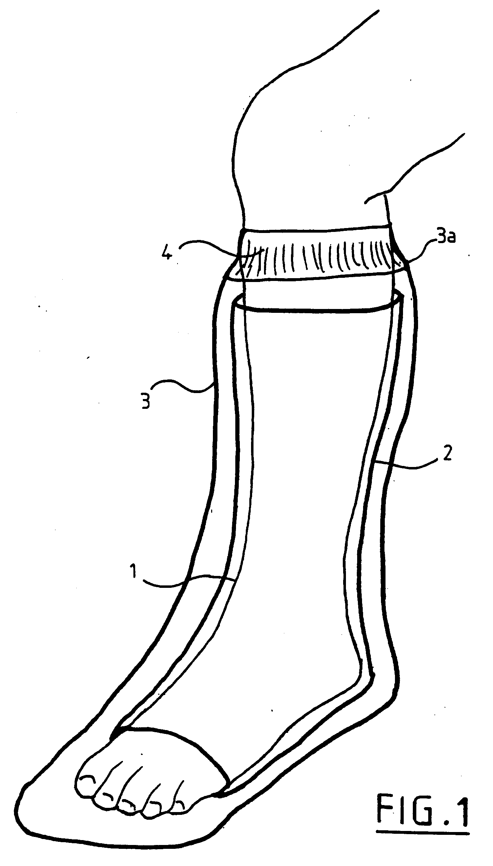

Le dispositif de protection représenté à la Fig. 1 est enfilé sur une jambe 1 qui porte un plâtre 2. De manière générale, un dispositif selon l'invention est destiné à être enfilé sur un membre qui porte un système de contention, tel qu'un plâtre, un pansement, etc., à protéger.

Le dispositif de protection représenté comprend un manchon 3 qui est ouvert à son extrémité haute 3a. Le manchon 3 est en matériau non mouillable tissé ou non-tissé ou en matière plastique, telle que du polyéthylène, polyuréthanne, etc.

De manière générale, le manchon 3 est ouvert à une seulement ou à ses deux extrémités. Il est adapté à la forme du membre et est prévu pour recouvrir le moyen de contention, le plâtre ou le pansement.

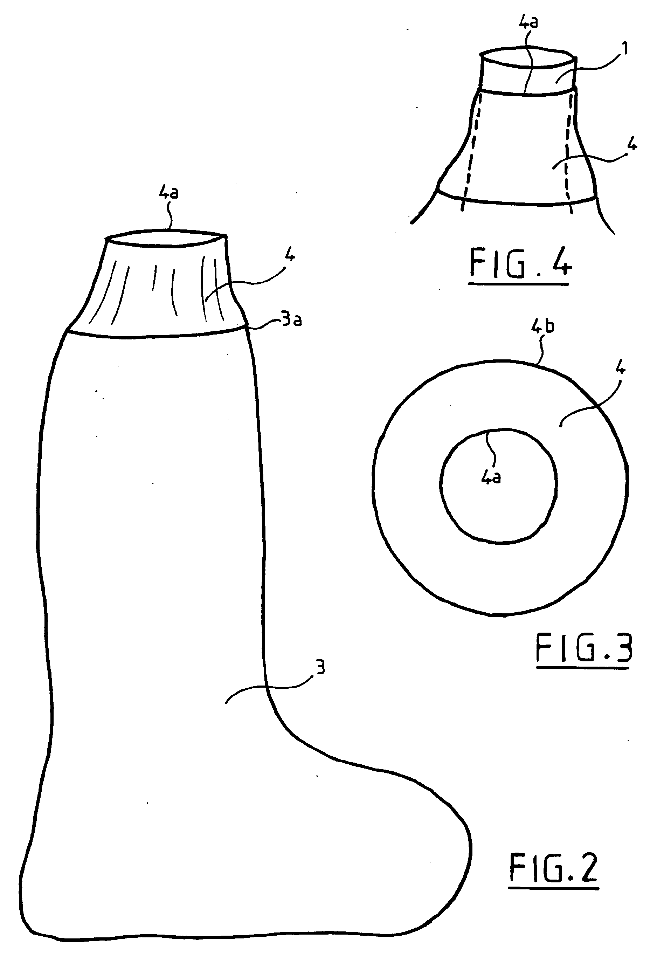

Le manchon 3 est pourvu, en prolongement de son ou de ses extrémités ouvertes, de moyens d'application 4 sur le membre. Ces moyens sont selon l'invention constitués d'un anneau élastique 4 en forme de couronne (Fig. 3) dont le diamètre de l'ouverture interne 4a est inférieur à celui du membre 1 à protéger. Ce diamètre est calculé de façon à ce que la partie interne de l'anneau 4 s'applique tangentiellement sur la peau saine du membre qui porte le moyen de contention comme cela est représenté à la Fig. 4. Ainsi, contrairement aux dispositifs de protection connus, le dispositif de l'invention est sans risque de compression ou gêne au retour veineux qui constitue l'effet de garrot.

Dans certains cas, le manchon 3 est ouvert aux deux extrémités, par exemple pour des traumatismes du genou. Un anneau élastique 4 est alors fixé à chaque extrémité du manchon 3.

L'anneau élastique ou chaque anneau 4 est avantageusement en latex.

L'anneau ou chaque anneau 4 est fixé à l'extrémité ou à chaque extrémité du manchon 3 de manière à ce que la protection soit étanche aux liquides. Il peut être soudé, collé ou cousu sur le manchon 3.

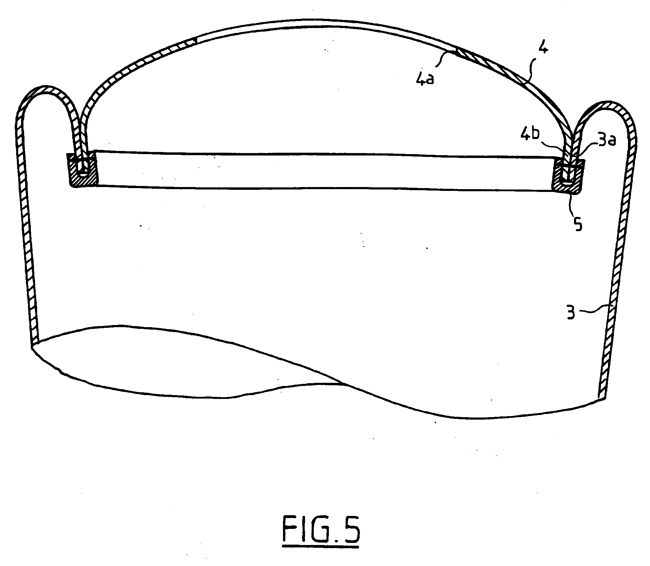

On a représenté à la Fig. 5 un mode de réalisation préféré de montage de l'anneau 4 sur le manchon 3. Sur cette Fig. 5, on voit le manchon 3 dont l'extrémité est repliée sur elle-même sur laquelle est monté l'anneau élastique 4. On voit également l'ouverture interne 4a de l'anneau 4. La partie externe 4b de l'anneau 4 et la partie d'extrémité 3a ouverte du manchon 3 sont montées bord à bord, sont recouvertes d'un ruban 5 de section en forme de "U". La partie 4b, l'extrémité 3a et le ruban 5 sont solidarisés par couture, ou surpiquage.

On remarquera que la couture est à l'intérieur du manchon 3.

## Revendications

1. Dispositif de protection étanche pour un moyen de contention, tel qu'un pansement ou un plâtre, porté par un membre (1) supérieur ou inférieur, dispositif comportant un manchon (3) qui est destiné à être enfilé sur ledit membre (1) et donc à le protéger, ledit dispositif étant prévu pour être appliqué d'une manière élastique sur ledit membre (1) par sa ou ses extrémités ouvertes (4a), ladite ou lesdites extrémités ouvertes (4a) présentant un diamètre inférieur à celui dudit membre (1), caractérisé en ce que ledit dispositif comporte au moins un anneau élastique (4) en forme de couronne, ledit ou chaque anneau (4) étant solidarisé par son extrémité externe (4b) par rapport audit membre (1) avec ledit manchon (3), ledit ou chaque anneau (4) présentant, d'une part, un diamètre maximal au niveau de ladite extrémité externe (4b) et, d'autre part, un diamètre minimal au niveau de son extrémité interne (4a) par rapport audit membre (1), ladite extrémité interne (4a) formant ladite ou chaque extrémité ouverte (4a) dudit dispositif.

2. Dispositif de protection étanche selon la revendication 1, caractérisé en ce que ledit ou chaque anneau élastique (4) est cousu sur une extrémité ouverte (3a) dudit manchon (3).

3. Dispositif de protection étanche selon la revendication 1, caractérisé en ce que ledit ou chaque anneau élastique (4) est collé ou soudé sur une extrémité ouverte (3a) dudit manchon (3).

4. Dispositif de protection étanche selon une des revendications précédentes, caractérisé en ce que ladite extrémité externe (4b) dudit ou de chaque anneau élastique (4) et une extrémité ouverte (3a) dudit manchon (3) sont montées bord à bord et sont recouvertes d'un ruban (5) de section en forme de "U".

5. Dispositif de protection étanche selon la revendication 4, caractérisé en ce que ladite extrémité externe (4b) dudit ou de chaque anneau élastique (4), ladite extrémité ouverte (3a) dudit manchon (3) et ledit ruban (5) sont solidarisés par couture ou surpiquage.

6. Dispositif de protection étanche selon la revendication 4, caractérisé en ce que ladite extrémité externe (4b) dudit ou de chaque anneau élastique (4), ladite extrémité ouverte (3a) dudit manchon (3) et ledit ruban (5) sont solidarisés par collage ou soudage.

7. Dispositif de protection étanche selon une des revendications précédentes, caractérisé en ce que ledit ou chaque anneau élastique (4) est en latex.

8. Dispositif de protection étanche selon une des revendications précédentes, caractérisé en ce que ledit manchon (3) est constitué d'une enveloppe en matière plastique.

9. Dispositif de protection étanche selon une des revendications 1 à 7, caractérisé en ce que ledit manchon (3) est constitué d'une enveloppe en matériau tissé ou non tissé.

## Claims

1. Watertight protection device for a means of support, such as a dressing or a plaster cast, borne on an upper or lower limb (1), a device consisting of a sleeve (3) which is intended to be slid over the said limb (1) and therefore to protect it, the said device being intended to be applied in an elastic manner over the said limb (1) by its open extremity or extremities (4a), the said open extremity or extremities (4a) having a diameter less than that of the aforesaid limb (1), characterised in that the said device consists of at least one elastic ring (4) in the shape of a crown (4), one or each ring (4) being brought together by its external extremity (4b) in relation to the said limb (1) with the said sleeve (3), the said or each ring (4) presenting, on the one hand, a maximum diameter at the level of the said external extremity (4b) and, on the other hand, a minimum diameter at the level of its internal extremity (4a) in relation to the said limb (1), the said internal extremity (4a) forming the said open extremity or extremities (4a) of the said device.

2. Watertight protection device in accordance with claim 1, characterised in that the said or each elastic ring (4) is sown to an open extremity (3a) of the said sleeve (3).

3. Watertight protection device in accordance with claim 1, characterised in that the said or each elastic ring (4) is glued or welded to an open extremity (3a) of the said sleeve (3).

4. Watertight protection device according to one of the preceding claims, characterised in that the said external extremity (4b) of the said or each elastic ring (4) and an open extremity (3a) of the said sleeve (3) are assembled side by side and are covered with a tape (5) "U" shaped in section.

5. Watertight protection device in accordance with claim 4, characterised in that the said external extremity (4b) of the said elastic ring or each elastic ring (4) the said open extremity (3a) of the said sleeve (3) and the said tape (5) are brought together by means of sewing or topstitching.

6. Watertight protection device in accordance with claim 4, characterised in that the said external extremity (4b) of the said elastic ring or each elastic ring (4) the said open extremity (3a) of the said sleeve (3) and the said tape (5) are brought together by means of gluing or welding.

7. Watertight protection device according to one of the preceding claims, characterised in that the said elastic ring or each ring (4) is made of latex.

8. Watertight protection device according to one of the preceding claims, characterised in that the said sleeve (3) is made of a covering of plastic material.

9. Watertight protection device according to one of claims 1 to 7, characterised in that the said sleeve (3) is made of a woven or unwoven material.

## Patentansprüche

1. Dichte Schutzvorrichtung für einen Stützverband, wie einen Gips oder anderen Verband, der von einer oberen oder unteren Gliedmaße (1) getragen wird, mit einer eine Hülse (3) umfassenden Einrichtung, die dazu bestimmt ist, auf die Gliedmaße (1) gestreift zu werden und sie somit zu schützen, sowie vorgesehen ist, mit ihrem offenen Ende oder ihren offenen Enden (4a) elastisch an der Gliedmaße (1) befestigt zu werden, wobei das offene Ende oder die offenen Enden (4a) einen kleineren Durchmesser als die Gliedmaße (1) aufweist bzw. aufweisen, **dadurch gekennzeichnet**, dass die Vorrichtung mindestens einen kranzförmigen, elastischen Ring (4) umfasst, der oder jeder Ring (4) mit seinem äußeren Ende (4b) bezüglich der Gliedmaße (1) fest mit der Hülse (3) verbunden ist, der oder jeder Ring (4) einerseits einen größten Durchmeser in Höhe des äußeren Endes (4b) und andererseits einen kleinsten Durchmesser nur in Höhe seines inneren Endes (4a) bezüglich der Gliedmaße (1) aufweist, und das innere Ende (4a) das oder jedes offene Ende (4a) der Vorrichtung bildet.

2. Dichte Schutzvorrichtung nach Anspruch 1, **dadurch gekennzeichnet,** dass der oder jeder elastische Ring (4) an ein offenes Ende (3a) der Hülse (3) angenäht ist.

3. Dichte Schutzvorrichtung nach Anspruch 1, **dadurch gekennzeichnet**, dass der oder jeder elastische Ring (4) an ein offenes Ende (3a) der Hülse (3) angeklebt oder angeschweißt ist.

4. Dichte Schutzvorrichtung nach einem der vorangehenden Ansprüche, **dadurch gekennzeichnet**, dass das äußere Ende (4ba) des oder jedes elastischen Rings (4) und ein offenes Ende (3a) der Hülse (3) mit ihren Rändern aneinander befestigt und mit einem Band (5) bedeckt sind, dessen Querschnitt U-förmig ist.

5. Dichte Schutzvorrichtung nach Anspruch 4, **dadurch gekennzeichnet**, dass das äußere Ende (4b) des oder jedes elastischen Rings (4), das offene Ende (3a) der Hülse (3) und das Band (5) durch Nähen oder Anheften fest miteinander verbunden sind.

6. Dichte Schutzvorrichtung nach Anspruch 4, **dadurch gekennzeichnet**, dass das äußere Ende (4b) des oder jedes elastischen Rings (4), das offene Ende (3a) der Hülse (3) und das Band (5) durch Kleben oder Schweißen fest miteinander verbunden sind.

7. Dichte Schutzvorrichtung nach einem der vorstehenden Ansprüche, **dadurch gekennzeichnet**, dass der oder jeder elastische Ring (4) aus Latex besteht.

8. Dichte Schutzvorrichtung nach einem der vorstehenden Ansprüche, **dadurch gekennzeichnet**, dass die Hülse (3) aus einer Kunststoffhülle besteht.

9. Dichte Schutzvorrichtung nach einem der Ansprüche 1 bis 7, **dadurch gekennzeichnet**, dass die Hülse (3) aus einer Hülle aus gewebtem oder nichtgewebtem Material besteht.
